# EUROPEAN PATENT APPLICATION

(11) **EP 2 096 113 A1**
(43) Date of publication of application: **02.09.2009**
(21) Application number: 08151067.9
(22) Date of filing: 05.02.2008
(51) Int. Cl.: C07D 491/22, A61K 31/4745, A61P 35/00

(54) **9-substituted camptothecin derivatives as antitumor compounds**

(71) Applicant: SIGMA-TAU Industrie Farmaceutiche Riunite S.p.A., 00144 Roma (IT); ISTITUTO NAZIONALE PER LO STUDIO E LA CURA DEI TUMORI, I-20133 Milano (IT)
(72) Inventor: Dallavalle Sabrina, 20059 Vimercate (IT); Merlini Lucio, 20122 Milan (IT); Zunino Franco, 20127 Milan (IT); Giannini Guiseppe, 00040 Pomezia (IT)
(74) Representative: Tagliafico, Giulia

(57) **Abstract**

Compounds are described with the general formula 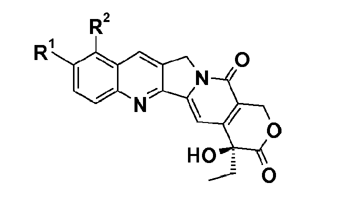 in which the groups are as defined in the description and characterized by the presence of substituents in position 9. Said compounds are endowed with potent topoisomerase I inhibiting activity and therefore are useful as medicines for the treatment of tumours and viral and parasite infections.

## Description

### FIELD OF THE INVENTION

The invention described herein relates to compounds useful as medicines, and particularly to camptothecin derivatives with substituents in position C-9, to procedures for their preparation, to their use as active agents with topoisomerase I inhibiting activity and to pharmaceutical compositions containing them as active ingredients.

### BACKGROUND TO THE INVENTION

Camptothecin is an alkaloid isolated by Wall et al. [J. Am. Chem. Soc., 1966, 88, 3888-3890] for the first time from the tree Camptotheca acuminata, native to China and belonging to the Nyssaceae family.

The molecule consists of a pentacyclic structure with a lactone in ring E, which is essential for cytotoxicity.

For a review of the camptothecins and the problems relating to their use as medicines as well as the solving of such problems, the reader is referred to EP 1 044 977, filed in the name of the present applicant.

Most of the camptothecins reported in the literature and tested as antitumor compounds are substituted in position 7. The patent and scientific literature reports also a few examples of camptothecins substituted in position 9, most relevant being 10-hydroxy-9-dimethylaminomethylcamptothecin (Topotecan), a drug in current clinical use, 9-nitrocamptothecin (rubitecan) and 9-aminocamptothecin, which have been submitted to preclinical and clinical studies.

### DESCRIPTION OF THE INVENTION

It has now been found that camptothecins substituted in position 9 by means of a hydroxymethyl or formyl or oxyiminomethyl or cyano groups, display substantial anticancer activity. This anticancer potency is comparable to that of compounds currently used in oncological clinical practice, and therefore the derivatives which are the subject of the present invention may make a major contribution to enriching the armamentarium available for the fight against cancer.

The compounds which are the subject of the present invention have the following general formula (I): 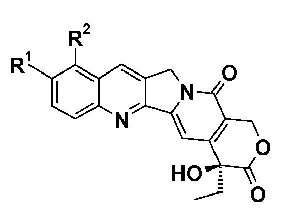 in which
R¹ is H, OH or O-alkyl;
R² is CH₂OalkylOalkylOalkyl, CN or CH=NOR₃, provided that when R² is CN, R¹ is not H or OH;
R³ is H, alkyl optionally substituted with OH, OCH₃, NH₂, COH, COOH, COOalkyl, aryl, arylkyl, said aryl or arylkyl being optionally substituted with OH, halo, NH₂, NHalkyl, N(alkyl)₂, a heteroyclic or a heterocyclo-(C₁-C₈) linear or branched alkyl group.
their N1-oxides, racemic mixtures, their individual enantiomers, their individual diastereoisomers, the E and Z forms, their mixtures, and pharmaceutically acceptable salts.

A further object of the present invention consists in the use of compounds with the above-mentioned general formula (I) as active ingredients for medicines, particularly for medicines useful as topoisomerase I inhibitors. Among the therapeutic applications deriving from the topoisomerase inhibiting activity we should mention tumours and viral and parasite infections.

Another object of the present invention consists in pharmaceutical compositions containing formula (I) compounds as active ingredients, in mixtures with pharmaceutically acceptable vehicles and excipients.

A first group of particularly preferred compounds consists of formula (I) compounds:
- 9-methoxyethoxymethoxymethylcamptothecin
- 9-methoxyiminomethylcamptothecin
- 9-benzyloxyiminomethylcamptotecin
- 9-tert-butoxyiminomethylcamptothecin
- 9-(2-amino)ethoxyiminomethylcamptothecin
- 9-(3-amino)propoxyiminomethylcamptothecin
- 10-hydroxy-9-tert-butoxyiminomethylcamptothecin
- 10-methoxy-9-tert-butoxyiminomethylcamptothecin
- 10-Methoxy-9-cyanocamptothecin
- 10-methoxy-9-formylcamptothecin

The compounds described in the present invention are topoisomerase I inhibitors and are therefore useful as medicines, particularly for the treatment of diseases that benefit from inhibition of said topoisomerase. In particular, the compounds according to the present invention display antiproliferative activity, and are therefore used for their therapeutic activity, and possess physicochemical properties which make them suitable for formulation in pharmaceutical compositions.

The pharmaceutical compositions contain at least one formula (I) compound as the active ingredient, in an amount such as to produce a significant therapeutic effect. The compositions covered by the present invention are entirely conventional and are obtained by using methods which are common practice in the pharmaceutical industry. According to the administration routes opted for, the compositions will be in solid or liquid form, suitable for oral, parenteral or intravenous administration. The compositions according to the present invention contain, along with the active ingredient, at least one pharmaceutically acceptable vehicle or excipient. These may be particularly useful coadjuvants, for example, solubilising agents, dispersion agents, suspension agents, or emulsifying agents.

Formula (I) compounds can also be used in combination with other active ingredients, for example, anticancer drugs or other drugs with antiparasite or antiviral activity, both in separate forms and in single-dosage forms.

The compounds according to the present invention are useful as medicines with anticancer activity, for example in lung cancers such as non-microcytoma lung cancer, or in colorectal and prostate cancer and glioma.

The compounds of formula I can be prepared from 10-hydroxy-20S-camptothecin, by a formylation reaction, for example the Duff reaction, to obtain 10-hydroxy-9-formylcamptothecin, as disclosed by W.D. Kingsbury, et al., J. Med. Chem. 1991, 34, 98-107.

Said 10-hydroxy-9-formylcamptothecin can be transformed in compounds of formula I where R¹ = OCH₃, by methylation of the OH group, and further elaboration of the formyl group.

Alternatively, said 10-hydroxy-9-formylcamptothecin can be transformed in the corresponding 10-trifluoromethanesulfonate or 10-toluenesulfonate, that in turn can be treated in the presence of a Pd catalyst to give 9-formylcamptothecin.

Compounds of formula I where R¹ =H can be prepared from the said 9-formylcamptothecin by reaction with compounds of formula R³ONH₂ or their salts, wherein R³ is as above defined. The reaction can be carried out by conventional methods well known to the person skilled in the art. The reaction is carried out in the presence of a base, for example an organic base, such as pyridine or triethylamine, using polar solvents, preferaby methanol or ethanol, at a temperature comprised between the room temperature and the boiling point of the solvent.

Alternatively, compounds of formula I where R¹=H can be prepared by Friedländer condensation of a compound of formula II with the reactant of formula III, according to the method reported by A. Ejima et al., J. Chem. Soc. Perkin Trans. I 1990, 27-31.

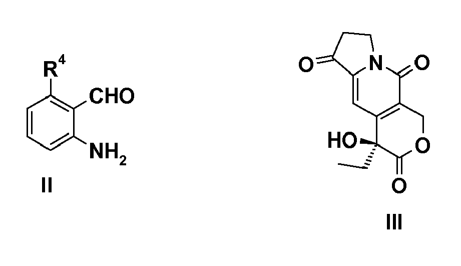

In compounds of formula II, R⁴ can be CHO, or CH₂OH, or a protected CHO group (for example 2-dioxolane or 2-dioxane or a 2-thiane), or protected CH₂OH group (for example a tetrahydropyranylether or an alkoxymethylether, or an alkoxyalkoxymethylether), or anotherCHO precursor well known to those skilled in the art (for example methyl, vinyl) suitable to be converted into a CHO group after the Friedländer condensation with the reagent of formula III

Compounds of formula II have not been prepared before, and the method of their synthesis is part of the present invention.
A preferred compound of formula II, where R⁴ is CH₃OCH₂CH₂OCH₂O, can be prepared as described in Scheme I:

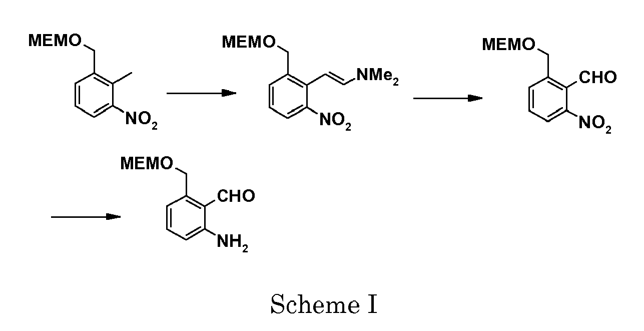

The following examples further illustrate the invention.

### Example 1

### 2-(2-Methoxyethoxymethoxymethyl)-6-nitro-benzaldehyde

A solution of 1-(2-methoxyethoxymethoxymethyl)-2-methyl-3-nitrobenzene (Shin, C.; et al., Heterocycles, 1996, 43, 891-898) (1.34 g, 5.25 mmol) in DMF (15 ml) was stirred with N,N-dimethylformamide dimethylacetal (1.1 ml, 7.88 mmol) and pyrrolidine (0.9 ml, 10.50 mmol) under N₂ for 2 h at 135°C. 30 ml of water were added and the resulting suspension was extracted with EtOAc. After drying and evaporation, the crude red oil (1.6 g) of {2-[2-(2-methoxyethoxymethoxymethyl)-6-nitro-phenyl]-vinyl}-dimethyl-amine, ¹H NMR (CDCl₃) δ: 2.90 (s, 3H, N-Me); 2.97 (s, 3H, N-Me); 3.42 (s, 3H, -OMe); 3.59 (t, 2H, J = 4.84 Hz, -CH₂-O); 3.77 (t, 2H, J = 4.84 Hz, -CH₂-O); 4.68 (s, 2H, OCH₂Ph); 4.86 (s, 2H, OCH₂O); 5.11 (d, 1H, CH=, J = 13.77 Hz); 6.74 (d, 1H, CH=, J = 13.77 Hz); 7.08 (dd, 1H, 1Ar, J = 7.82, 7.82 Hz); 7.52 (d, 1H, 1Ar, J = 7.82 Hz); 7.55 (d, 1H, 1Ar, J = 7.82 Hz). was utilized for the next reaction without further purification.
A mixture of the said red oil and sodium periodate (3.3 g, 15.48 mmol) was stirred in 50% aqueous THF (60 ml) at room temperature for 3 h. The insoluble was removed by filtration and the filtrate diluted with EtOAc (20 ml). The organic layer was washed with water and dried. The solvent was removed under reduce pressure, and the residue was purified by flash chromatography (EtOAc/hexane: 1:3) to give 2-(2-methoxyethoxymethoxymethyl)-6-nitro-benzaldehyde (1.20 g), ¹H NMR (CDCl₃) δ: 3.24 (s, 3H, -OMe); 3.44 (t, 2H, J = 5.21 Hz, -CH₂-O); 3.60 (t, 2H, J = 5.21 Hz, -CH₂-O); 4.72 (s, 2H, OCH₂Ph); 4.76 (s, 2H, OCH₂O); 7.81 (dd, 1H, 1Ar, J = 8.19, 8.19 Hz); 7.95 (d, 1H, 1Ar, J = 8.19 Hz); 8.11 (d, 1H, 1Ar, J = 8.19 Hz).

### Example 2

### 2-Amino-6-(2-methoxyethoxymethoxymethyl)-benzaldehyde

A suspension of 2-(2-methoxyethoxymethoxymethyl)-6-nitrobenzaldehyde (1.2 g, 4.46 mmol) and 10% Pd/C (120 mg) in EtOH (100 ml) was hydrogenated at atmospheric pressure until the uptake of hydrogen was complete. The catalyst was filtered through Celite and washed with ethanol. Evaporation gave the title compound, ¹H NMR (CDCl₃) δ: 3.32 (s, 3H, -OMe); 3.35-4.84 (m, 4H, -CH₂-O); 4.69 (s, 2H, OCH₂Ph); 4.77 (s, 2H, OCH₂O); 6.53 (d, 1H, 1Ar, J = 7.07 Hz); 7.75 (d, 1H, 1Ar, J = 8.56 Hz); 7.23 (dd, 1H, 1Ar, J = 7.07, 8.56 Hz); 7.39 (brs, 2H, NH₂).

### Example 3

### 9-(2-Methoxyethoxymethoxymethyl)camptothecin

The ketone of formula III (990 mg, 3.74 mmol), ZnCl₂ (3.8 g, 28.08 mmol) and molecular sieves were added to a solution of 2-amino-6-(2-methoxyethoxymethoxymethyl)-benzaldehyde in EtOH and the mixture was heated at 70°C under an atmosphere of nitrogen for 5 h. Ethanol was removed in vacuo and the residue was purified by flash chromatography with CH₂Cl₂/CH₃OH 97:3 to give 9-(2-methoxyethoxymethoxymethyl)camptothecin as a white solid. Yield 830 mg (40%), ¹H NMR (CDCl₃) δ: 1.06 (t, 3H, J= 7.44 Hz, -CH₃); 1.79-1.99 (m, 2H, -CH₂); 3.43 (s, 3H, -OMe); 3.59 (t, 2H, -CH₂O-, J = 4.84 Hz); 3.75 (t, 2H, -CH₂O-, J = 4.84 Hz); 4.85 (s, 2H, -OCH₂Ph-); 5.10 (s, 2H, -OCH₂O-); 5.28, 5.74 (AB, -CH₂); 5.31 (2H, s, -CH₂); 7.25 (s, 1H, 1Ar); 7.67(d, 1H, 1Ar, J = 8.19 Hz); 7.76 (dd, 1H, 1Ar, J = 8.19, 8.19 Hz); 8.19 (d, 1H, 1Ar, J = 8.19 Hz); 8.75 (s, 1H, H-7).

### Example 4

### 9-tert-Butoxyiminomethylcamptothecin

A suspension of 9-formylcamptothecin (50 mg, 0.132 mmol) in 10 ml of EtOH was added to a solution of O-tert-butylhydroxylamine hydrochloride (50 mg, 0.396 mmol) in 1.5 ml of pyridine and refluxed for 2 h. After evaporation and chromatography with CH₂Cl₂/ MeOH 97:3, 9-tert-butoxyiminomethylcamptothecin was obtained as a yellow solid (20 mg, 35% yield). ¹H NMR (CDCl₃) δ: 1.04 (t, 3H, J= 7.44 Hz, -CH₃); 1.44 (s, 9H, tBu); 1.80-1.95 (m, 2H, -CH₂); 3.75 (s, 1H, -OH); 5.30, 5.75 (AB, CH₂); 5.31 (2H, s, -CH₂); 7.67 (s, 1H, 1Ar); 7.70-7.85 (m, 2H, 2Ar); 8.21 (d, 1H, 1Ar, J = 8.19 Hz); 8.56 (s, 1H, H-7); 9.50 (s, 1H, CH=).

### Example 5

### 9-(2-Aminoethoxy)iminomethylcamptothecin

A suspension of 9-formylcamptothecin (50 mg, 0.132 mmol) in 10 ml of EtOH was treated with 1.5 ml of pyridine and O-(2-aminoethyl)-hydroxylamine hydrochloride (45 mg, 0.398 mmol) and refluxed for 2 h. Evaporation and chromatography with CH₂Cl₂/ MeOH 8:2 gave 9-(2-aminoethoxy)iminomethylcamptothecin as a yellow solid (23 mg, 40% yield).
¹H NMR (DMSO-d₆) δ: 0.89 (t, 3H, J= 7.44 Hz, -CH₃); 1.82-1.94 (m, 2H, - CH₂); 3.20 (t, 2H, -CH₂NH₂, J = 5.95 Hz); 4.41 (t, 2H, -CH₂O-, J = 5.95 Hz); 5.33 (2H, s, -CH₂); 5.44 (2H, s, -CH₂); 6.55 (s, 1H, -OH); 7.37 (s, 1H, H-14); 7.60 (brs, 2H, -NH₂); 7.93 (dd, 1H, 1Ar, J = 8.19, 8.19 Hz); 8.02 (d, 1H, 1Ar, J = 8.19 Hz); 8.27 (d, 1H, 1Ar, J = 8.19 Hz); 8.95 (s, 1H, H-7); 9.45 (s, 1H, CH=).

### Example 6

### 10-hydroxy-9-tert-butoxylmlnomethylcamptothecin

10-Hydroxy-9-formylcamptothecin (28 mg) in 6 ml of MeOH was treated with 27 mg of NH₂OH.HCl, and 8 mg of NaOH, and the mixture left overnight at room T. Evaporation of the solvent, taking up with CH₂Cl₂/MeOH and chromatography on silica gel with CH₂Cl₂:MeOH 98:2 as eluent gave 25 mg of 10-hydroxy-9-tert-butoxyiminomethylcamptothecin, mass m/z = 463, ¹H NMR (DMSO-d₆) δ: 0.85 (t, 3H, -CH₃); 1.40 (s, 9H, tBu); 1.8-1.9 (m, 2H, - CH₂); 5.32 (s, 2H, H2-17), 5.40 (s, 2H, H2-5, CH₂); 6.52 (OH): 7.27 (s, 1H, H-14); 7.56 (d, 1H, H-12, J = 8 Hz); 8.12 (s, 1H, H-11, J=8 Hz); 8.90 (s, 1H, H-7); 9.38 (s, 1H, CH=).

### Example 7

### 10-methoxy-9-tert-butoxyiminomethylcamptothecin

10-hydroxy-9-tert-butoxyiminomethylcamptothecin (26 mg ) in 10 ml MeOH was treated with a solution of CH₂N₂ in ether, and the mixture left overnight at room T. Adding acetic acid and evaporation gave 14 mg of 10-methoxy-9-tert-butoxyiminomethylcamptothecin ¹H NMR (DMSO-d₆) δ: 0.87 (t, 3H, -CH₃); 1.40 (s, 9H, tBu); 1.8-1.9 (m, 2H, -CH₂); 4.03 (s, 3H, OMe); 5.32 (s, 2H, H2-17), 5.42 (s, 2H, H2-5, CH₂); 6.50 (OH): 7.28 (s, 1H, H-14); 7.85 (d, 1H, H-12, J = 8 Hz); 8.25 (s, 1H, H-11, J=8 Hz); 8.65 (s, 1H, H-7); 9.37 (s, 1H, CH=).

### Example 8

### 10-Methoxy-9-cyanocamptothecin

To a solution of 10-hydroxy-9-formylcamptothecin (44 mg, 0.112 mmol) in 15 ml of formic acid, hydroxylamine hydrochloride (20.3 mg, 0.29 mmol) and sodium formate (99 mg, 1.45 mmol) were added. The resulting solution was refluxed for 6 h. The mixture was concentrated in vacuo and the residue was purified by flash chromatography with CH₂Cl₂/CH₃OH 98:2 to give 10-hydroxy-9-cyanocamptothecin as a yellow solid. Yield 29 mg.
The crude product was suspended in 5 ml of MeOH and treated with an ethereal solution of CH₂N₂. Adding AcOH and evaporation gave 10-methoxy-9-cyanocamptothecin, ¹H NMR (DMSO-d₆) δ: 0.84 (t, 3H, J= 6.70 Hz, -CH₃); 1.8-1.9 (m, 2H, -CH₂); 4.12 (s, 3H, OMe); 5.30 (2H, s, -CH₂); 5.42 (2H, s, -CH₂); 6.45 (s, 1H, -OH); 7.30 (s, 1H, H-14); 7.90 (d, 1H, 1Ar, J = 8.9 Hz); 8.49 (d, 1H, 1Ar, J = 8.93 Hz); 8.71 (s, 1H, H-7).

### Cytotoxic activity on NCI-H460 cells

NCI-H460 non-microcytoma lung cancer cells were kept in RPMI 1640 culture medium containing 10% FCS and 1% glutamine. The cytotoxicity test to analyse the activity of the molecules was performed as follows. The cells were seeded in a volume of 250 µl in 96-well plates and incubated for 2 hours at 37°C with scalar concentrations of the products in a humidified atmosphere containing 5% CO₂. At the end of the incubation, the molecules were removed by overturning the plates and adding sterile buffered saline solution (PBS) three times. The RPMI 1640 culture medium containing 10% FCS (200 µl) was added to the wells and the plates were incubated for another 72 hours. At the end of the incubation, the plates were overturned again and dried on paper, before adding 200 µl of PBS and 50 µl of 80% TCA. The plates were incubated again in ice for at least 1 hour. The TCA was removed by overturning the plates and the plates were first dried on paper and then washed three times by immersion in distilled water and overturning. The plates were dried first on paper and then in a thermostatically regulated incubator at 60°C for 10 min. 200 µl of 0.4% sulforodamine B in 1% acetic acid were added to all wells. The plates were incubated at room temperature for another 30 min. The sulforodamine B was removed by overturning, the plates were washed by immersion in 1% acetic acid three times and then dried first on blotting paper and then in the thermostat at 60°C for 10 min. Lastly, 200 µl of Tris base 10 mM were added to all wells and the plates were subjected to stirring for at least 20 min. The optical density was measured with a Multiskan spectrophotometer at 540 nm. Incubation with the products was capable of inhibiting proliferation in a concentration-dependent manner. Table 1 presents the IC₅₀ values (product concentration that inhibits cell survival by 50%]. IC₅₀ values for topotecan have also been reported for comparison purposes (Dallavalle S. et al., J. Med. Chem., 44, 3264-74, 2001).

**Table 1.**

| Cytotoxicity of camptothecin derivatives | |
|---|---|
| Compound (CPT = camptothecin) | IC50 (µM) H-460 |
| CPT-9-CH=NOC(CH₃)₃ | 0.067 |
| CPT-9-CH=NOCH₂CH₂NH₂ | 2.53+0.1 |
| 10-OH-CPT-9-CH=NOC(CH₃)₃ | 0.35 |
| 10-OCH₃-CPT-9-CH=NOC(CH₃)₃ | 0.31 |
| 10-OCH₃-CPT-9-CN | 0.27 |
| topotecan (J. Med. Chem. 2001) | 1.38 |

## Claims

1. Compounds with general formula (I) 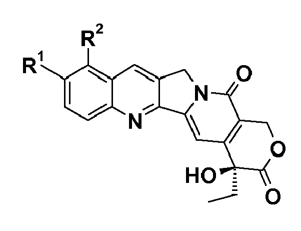 in which
R¹ is H, OH, O-alkyl
R² is CH₂OalkylOalkylOalkyl, CN, or CH=NOR³, provided that when R² is CN, R¹ is not H or OH;
R³ is H, aryl, alkyl optionally substituted with OH, OCH₃, NH₂, NH-alkyl, N(alkyl)₂, COH, COOalkyl, aryl optionally substituted with OH, halo, NH₂, NHalkyl, N(alkyl)₂,
the corresponding N1-oxides, racemic mixtures, their individual enantiomers, their individual diastereoisomers, the E and Z forms of the oximes, their mixtures, and pharmaceutically acceptable salts.

2. Compounds according to claim 1, selected from the group consisting of:
- 9-methoxyethoxymethoxymethylcamptothecin
- 9-methoxyiminomethylcamptothecin
- 9-benzyloxyiminomethylcamptothecin
- 9-tert-butoxyiminomethylcamptothecin
- 9-(2-amino)ethoxyiminomethylcamptothecin
- 9-(2-amino)propoxyiminomethylcamptothecin
- 10-methoxy-9-formylcamptothecin
- 10-hydroxy-9-tert-butoxyiminomethylcamptothecin
- 10-methoxy-9-tert-butoxyiminomethylcamptothecin
- 10-methoxy-9-cyanocamptothecin
- 10-methoxy-9-formylcamptothecin.

3. A pharmaceutical composition containing at least one compound according to claims 1-2 as the active ingredient in mixtures with at least one pharmaceutically acceptable vehicle and/or excipient.

4. A process for preparing the pharmaceutical composition according to claim 3, comprising mixing at least one of the compound according to clam 1 or 2 with at least one pharmaceutically acceptable vehicle and/or excipient.

5. Use of the compound according to claims 1-2 as medicines.

6. Use of the compound according to claims 1-2 for the preparation of a medicine with topoisomerase 1 inhibiting activity.

7. Use according to claim 6 for the preparation of a medicine with anticancer activity.

8. Use according to claim 6 for the preparation of a medicine with antiparasite activity.

9. Use according to claim 6 for the preparation of a medicine with antiviral activity.

10. A process for the preparation of the compound according to claim 1, which comprises reacting a compound of formula II 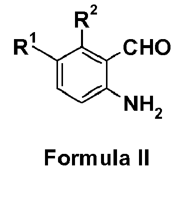 wherein R² is CHO or a CHO precursor, with (S)-9-ethyl-9-hydroxy-2,3,6,9-tetrahydro-7H-pyrrolo[1,2-b]isoquinoline-1,5,8-trione in the presence of ZnCl2 and molecular sieves.

11. A process for the preparation of compounds of formula II where R¹ is H which comprises the following steps:
a) reacting 1-(2-methoxyethoxymethoxymethyl)-2-methyl-3-nitrobenzene with N,N-dimethylformamide dimethylacetal and pyrrolidine;
b) treatment of the previous intermediate with sodium periodate
c) hydrogenating 2-(2-methoxyethoxymethoxymethyl)-6-nitrobenzaldehyde in the presence of 10% Pd/C at atmospheric pressure.
